Europäisches Patentamt

European Patent Office

Office européen des brevets

(11) Veröffentlichungsnummer: **0 144 588**
**B1**

(12) **EUROPÄISCHE PATENTSCHRIFT**

(45) Veröffentlichungstag der Patentschrift:
13.01.88

(51) Int. Cl.⁴: **A 61 F 2/34**

(21) Anmeldenummer: 84111616.3

(22) Anmeldetag: 28.09.84

(54) **Hüftgelenkspfanne.**

(30) Priorität: 12.12.83 CH 6615/83

(43) Veröffentlichungstag der Anmeldung:
19.06.85 Patentblatt 85/25

(45) Bekanntmachung des Hinweises auf die Patenterteilung:
13.01.88 Patentblatt 88/2

(84) Benannte Vertragsstaaten:
AT DE FR GB IT SE

(56) Entgegenhaltungen:
EP-A-0 063 706
FR-A-1 596 132
FR-A-2 329 249
FR-A-2 429 009
LU-A-77 518

(73) Patentinhaber: GEBRÜDER SULZER
AKTIENGESELLSCHAFT, Zürcherstrasse 9, CH-8401 Winterthur (CH)

(72) Erfinder: Frey, Otto, Wallrütistrasse 56, CH- 8404 Winterthur (CH)

(74) Vertreter: Dipl.- Ing. H. Marsch Dipl.- Ing. K. Sparing Dipl.- Phys.Dr. W.H. Röhl Patentanwälte, Rethelstrasse 123, D-4000 Düsseldorf (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Hüftgelenkspfanne zur zementfreien Verankerung im menschlichen Becken, bestehend aus einem mit Rippen und einem Schlitz versehenen Aussenring und aus einen in diesen eingelegten Pfannenkörper, welcher Aussenring mit einem inneren Hohlkonus für einen daran angepassten Konus des Pfannenkörpers versehen ist.

Bei Implantaten für den menschlichen Körper ist man ganz allgemein bestrebt, den Fremdkörper im Gewebe möglichst klein im Volumen zu halten. Dieses Bestreben führt bei künstlichen Gelenkpfannen zu relativ dünnwandigen Pfannenkörnern; da wegen der Gleiteigenschaften die Pfannenkörper sehr häufig aus Kunststoff, insbesondere aus Polyäthylen, hergestellt werden, sind die Pfannenkörper relativ nachgiebig und übertragen die Beckenbewegungen auf die zueinander kongruenten Gleitflächen. Diese Bewegungen führen zu dem sogenannten "Kirschkern-Effekt", worunter man das "Herausarbeiten" eines festen Kerns aus einem weicheren Material bei Relativbewegungen zueinander versteht. Sofern die Pfannenkörper aus Kunststoff bestehen, neigen sie unter den dauernden Belastungen weiterhin zu plastischen Verformungen, die zu Veränderungen ihrer Schalenfläche führen. Dadurch wird die Kongruenuz der eine Gleitfläche des Gelenks bildende Schalenoberfläche mit der anderen Gleitfläche, die von der Oberfläche des Gelenkkopfes gebildet wird, gestört, was zu erhöhtem Abrieb führt. Es besteht daher die Forderung, dass die Hüftgelenkspfanne fest und starr im Becken verankert sein muss. Diese Forderung hat zu einer Konstruktion geführt, bei der ein Pfannenkörper von einem Aussenring umgeben und in diesem unter Vorspannungen gehalten wird (DE-A-29 25 089). Damit ist zwar der Pfannenkörper gegen den direkten Einfluss der Beckenbewegungen geschützt; bei dieser Konstruktion treten aber beim plastischen Kaltfliessen eines Pfannenkörpers aus Kunststoff Relativbewegungen zwischen dem Pfannenkörper und seinem Aussenkörper auf, die zu Verschleiss und Abrieb führen, der zwischen die Gleitflächen des Gelenkes gelangen kann. Diese bekannte Pfanne ist zwar in sich steif und beispielsweise gegen Kippbewegungen, die sie als Ganzes belasten, stabil und widerstandsfähig, vermag jedoch elastischen Beckenbewegungen nicht zu folgen, die Aufweitungen und Kontraktionen des im Becken geschaffenen Pfannenhohlraumes bewirken. Bei dieser Konstruktion besteht daher für die Pfanne nach der Implantation keine Möglichkeit, sich zu "setzen", d.h. sich bei den genannten Bewegungen des Beckens und/oder bei plastischen Verformungen des sehr häufig aus Kunststoff bestehenden Pfannenkörpers selbstätig zu fixieren.

Eine Pfannenkonstruktion der eingangs genannten Art ist aus der EP-A-0 083 708 bekannt; bei dieser Konstruktion weist der Aussenring längs einer Mantellinie einen einzigen relativ breiten, durchgehenden Schlitz auf. Durch ein Einpressen des Pfannenkörpers wird der Aussenring aufgeweitet und in den Knochen "einge. pannt". Für das Becken ergeben sich dabei Belastungen, die über den Umfang des Ringes ungleichmässig verteilt sind, da die Kräfte im Schlitz und diametral dazu verschwinden, während sie in einem Winkel von 90° zu dem durch den Schlitz gehenden Durchmesser einen Maximalwert erreichen. Ursache für diese ungleichmässige Verteilung der Kräfte ist die "maulartige" Bewegung, die der Ring beim Aufweiten ausführt. Zwar gibt diese Pfannenkonstruktion den künstlichen Pfannenhohlraum erweiternden Bewegungen durch ein tieferes Eindringen des Pfannenkörpers in den Aussenring nach, so dass sich diese Pfanne "setzen", d.h. selbst fixieren kann; die ungleiche Verteilung der "Ringkräfte" über den Umfang bleibt jedoch erhalten.

Aufgabe der Erfindung ist es daher, eine Hüftgelenkspfanne der genannten Art zu schaffen, die sich selbst fixiert, falls es nach der Implantation während der anfänglichen "Betriebszeit" unter Belastung zu einem "Setzen" kommt, und bei der die Ringspannungen sich gleichmässig über den Umfang verteilen. Diese Aufgabe wird nach der Erfindung dadurch gelöst, dass über den Umfang verteilt und wechselseitig von beiden Ringseiten her, entlang Mantellinien verlaufende Schlitze im Aussenring vorgesehen sind, und dass die Tiefe der Schlitze geringer als die Mantelhöhe des Aussenrings ist.

Die Verteilung der Schlitze auf den ganzen Umfang und von beiden Ringseiten her ermöglicht ein Aufweiten des Aussenringes, das von der zentralen Achse im wesentlichen radial nach aussen erfolgt und eine gleichmässige Kräfteverteilung bewirkt. Dabei ergibt sich ein postoperatives Eindringen des Pfannenkörpers in den Aussenring, durch das eine Selbstfixierung dieses Körpers und der ganzen Pfanne erfolgt. Die Anordnung und Verteilung einer Vielzahl von Schlitzen verleiht dem Aussenring eine solche Elastizität, dass er den Beckenbewegungen zu folgen vermag, wobei ein Mitgehen mit Beckenkontraktionen im Rahmen der Elastizität des im Aussenring fixierten Pfannenkörpers erfolgen kann.

Die Haftung zwischen den Oberflächen der beiden Konen kann verbessert werden, wenn der innere Hohlkonus des Aussenrings und/oder der Konus des Pfannenkörpers mit einer Oberflächenstruktur versehen sind, wobei sich beispielsweise bewährt hat, wenn die Oberflächenstrukturen in ineinandergreifenden Verzahnungen bestehen.

Für das elastische Aufweiten des Aussenrings ist es weiterhin vorteilhaft, wenn die Tiefe der Schlitze mindestens das 0,5-fache der Mantelhöhe des Aussenringes beträgt.

Während - wie bereits mehrfach betont - der Pfannenkörper im allgemeinen aus Kunststoff besteht, ist das Material für den Ring bevorzugt ein geeignetes Metall, wobei alle in der Implantat-Technik üblichen Metalle oder Metallegierungen verwendet werden können.

Im folgenden wird die Erfindung anhand eines Ausführungsbeispiels im Zusammenhang mit der Zeichnung näher erläutert.

Fig. 1 zeigt teilweise im Schnitt eine Seitenansicht der neuen Hüftgelenkspfanne, während

Fig. 2 ein Detail von Fig. 1 in grösserem Maßstab ist.

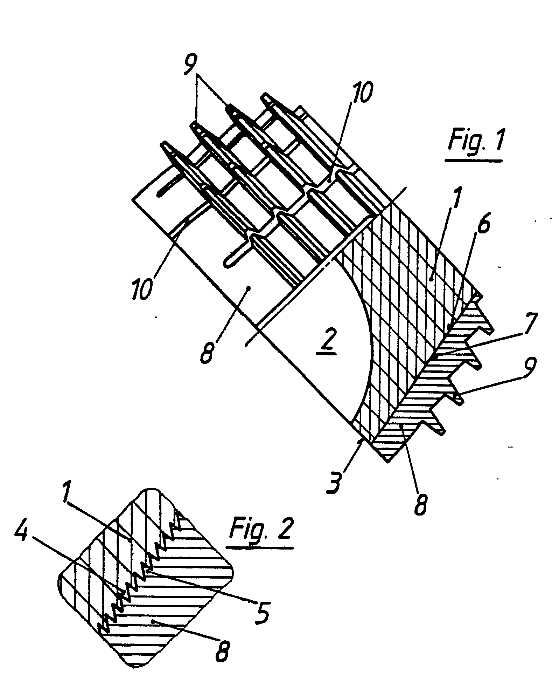

Die Hüftgelenkspfanne nach Fig. 1 hat einen Pfannenkörper 1, dessen Querschnitt ein gleichseitiges Trapez bildet, so dass seine Grundform ein Kegelstumpf ist. Von der breiten Basis 3 aus ist in den Pfannenkörper 1 die eigentliche Pfannenschale 2 in Form einer hohlen Halbkugel eingearbeitet, die den Gelenkkopf einer nicht dargestellten Femurkopfprothese aufnimmt.

Auf dem Konus 6 des Pfannenkörpers 1 sitzt ein innerer Hohlkonus oder Innenkonus 7 eines Metallrings 8, der auf seiner Aussenseite mit einer Verankerungsstruktur, beispielsweise mit Tragrippen 9 oder einem Schraubengewinde, versehen ist.

Der Konus 6 des Pfannenkörpers 1 und der Innenkonus 7 des Ringes 8 sind so aufeinander abgestimmt, dass sie im Passsitz ineinandergreifen. Wie Fig. 2 verdeutlicht, tragen die Oberflächen der Konen 6 und 7 Verzahnungen 4 und 5, die in Art eines "Positiv/negativ"-Profils ineinandergreifen, wobei die Abmessungen der Struktur jeweils etwa einige Zehntel mm betragen.

Von beiden Enden her sind im Mantel des Ringes 4 über den Umfang gleichmässig verteilt Schlitze 10 vorgesehen. Diese Schlitze 10, die von einem bzw. vom anderen Ende des Ringes 8 her alternierend aufeinander folgen, haben jeweils eine Tiefe, die mindestens bis zur Hälfte der Mantelhöhe des Ringes 8 reicht.

Bei der Implantation wird zunächst der Aussenring 8 in einen operativ vorbereiteten Hohlraum im Beckenknochen eingesetzt, z. B. eingepresst, eingeschlagen oder - wenn die Struktur 9 auf der Aussenseite des Ringes 8 in einem Gewinde besteht - eingeschraubt; anschliessend drückt der Operateur den Pfannenkörper 1 in den Innen- oder Hohlkonus 7, wobei die Verzahnungen 4 und 5 aneinandergreifen und einen sicheren Sitz des Pfannenkörpers 1 im Aussenring 8 gewährleisten.

## Patentansprüche

1. Hüftgelenkspfanne zur zementfreien Verankerung im menschlichen Becken, bestehend aus einem mit Rippen und einem Schlitz (10) versehenen Aussenring (8) und aus einem in diesen eingelegten Pfannenkörper (1), welcher Aussenring (8) mit einem inneren Hohlkonus (7) für einen daran angepassten Konus (6) des Pfannenkörpers (1) versehen ist, dadurch gekennzeichnet, dass über den Umfang verteilt und wechselseitig von beiden Ringseiten her, entlang Mantellinien verlaufend Schlitze (10) im Aussenring (8) vorgesehen sind, und dass die Tiefe der Schlitze (10) geringer als die Mantelhöhe des Aussenrings (8) ist.

2. Hüftgelenkspfanne nach Anspruch 1, dadurch gekennzeichnet, dass der innere Hohlkonus (7) des Aussenringes (8) und/oder der Konus (6) des Pfannenkörpers (1) mit einer Oberflächenstruktur versehen sind.

3. Hüftgelenkspfanne nach Anspruch 2, dadurch gekennzeichnet, dass die Oberflächenstrukturen in ineinandergreifenden Verzahnungen (4, 5) bestehen.

4. Hüftgelenkspfanne nach Anspruch 1, dadurch gekennzeichnet, dass die Tiefe der Schlitze (10) mindestens das 0,5-fache der Mantelhöhe des Aussenringes (8) beträgt.

## Claims

1. An acetabulum for cement-free fixing in the human pelvis and comprising an outer ring (8) formed with ribs and a slot (10) and a socket member (1) inserted in the ring, the same having an inner hollow cone (7) for a companion cone (6) of the socket member (1), characterised in that the outer ring (8) is formed with slots (10) which are distributed over its periphery and, starting alternately from both sides of the ring, extend along generatrices, and slot depth is less than the thickness of the shell or envelope of the outer ring (8).

2. An acetabulum according to claim 1, characterised in that the inner hollow come (7) of the outer ring (8) and/or the cone (6) of the socket member (1) have a surface structure.

3. An acetabulum according to claim 2, characterised in that the surface structures are toothings (4, 5) which engage one another.

4. An acetabulum according to claim 1, characterised in that the depth of the slot (10) is at least half the thickness of the shell or envelope of the outer ring (8).

## Revendications

1. Cuvette d'articulation coxofémorale pour l'ancrage sans ciment dans le bassin humain, comprenant une bague externe (8) munie de nervures et d'une fente (10), ainsi qu'un corps de cuvette (1) inséré dans cette bague, laquelle bague externe (8) est pourvue d'un cône creux intérieur (7) destiné à un cône (8) du corps de cuvette (1), qui lui est adapté, caractérisée par le fait que des fentes (10), prévues dans la bague externe (8), sont réparties sur le pourtour et s'étendent le long de génératrices, alternativement à partir des deux côtés de la bague; et par le fait que la profondeur des fentes (10) est plus faible que la hauteur de l'enveloppe de la bague externe (8).

2. Cuvette d'articulation coxofémorale selon la revendication 1, caractérisée par le fait que le cône creux intérieur (7) de la bague externe (8) et/ou le cône (6) du corps de cuvette (1) sont munis d'une structure superficielle.

3. Cuvette d'articulation coxofémorale selon la revendication 2, caractérisée par le fait que les structures superficielles sont constituées par des dentures (4, 5) imbriquées l'une dans l'autre.

4. Cuvette d'articulation coxofémorale selon la revendication 1, caractérisée par le fait que la profondeur des fentes (10) représente au moins 0,5 fois la hauteur de l'enveloppe de la bague externe (8).

0 144 588

Fig. 1

Fig. 2

1